# EUROPEAN PATENT SPECIFICATION

(11) **EP 4 431 011 B1**
(45) Date of publication and mention of the grant of the patent: **25.11.2026**
(21) Application number: 23192079.4
(22) Date of filing: 18.08.2023
(51) Int. Cl.: A61B 5/021, A61B 5/022

(54) **SPHYGMOMANOMETER**
SPHYGMOMANOMETER
SPHYGMOMANOMÈTRE

(30) Priority: 14.03.2023 CN 202320485287 U
(43) Date of publication of application: 18.09.2024
(73) Proprietor: Yasee Biomedical Co., Ltd., 266000 Shandong (CN)
(72) Inventor: HAN, Zhaowang, Shandong, 266000 (CN); CHEN, Xinming, Shandong, 266000 (CN)
(74) Representative: Eder Schieschke & Partner mbB

(56) References cited:
- CN-A- 103 735 258
- CN-A- 104 188 643
- JP-A- H01 236 034
- US-A- 4 178 918
- US-A1- 2005 187 484
- US-A1- 2007 167 845
- US-A1- 2008 146 947
- US-A1- 2010 010 356

## Description

### BACKGROUND OF THE INVENTION

### 1. Technical Field

The present application relates to medical instruments. More particularly, the present application relates to a sphygmomanometer, to a Bluetooth electronic sphygmomanometer, in particular to a sphygmomanometer based on a reversible air pump, and especially to an electronic sphygmomanometer using the MWI (measuring while inflating) method.

### 2. Description of Related Art

Most existing electronic sphygmomanometers work on the MWD (measuring while deflating) method for measurement of blood pressure values. The MWD method is about inflating a cuff with an air pump until the cuff compress the arm to the extent that it cuts the blood flow in the brachial artery, then gradually deflating the cuff, and pressure data are measured during the decompression process. Such an existing sphygmomanometer using the MWD method structurally needs an electromagnetic valve working with an air pump for inflating the cuff and a quick bleeding valve for deflating the cuff under control. Consequently, the known sphygmomanometer is packed with mechanical components and becomes structurally complicated. In particular, the electromagnetic valve and the bleeding valve together with their controlling units take considerable space in the interior of the sphygmomanometer, making microminiaturization of the sphygmomanometer difficult.

Also, most existing electronic sphygmomanometers are formed as a bulky box with a display attached thereto. Therefore, in order to allow such a sphygmomanometer to be affixed to the wrist or arm of a to-be-measured object stably, it is desired that the cuff connected to the main body of the sphygmomanometer has an increased width matching that of the main body. However, this unavoidably enlarges the sphygmomanometer and its fixtures in size, and in turn makes the sphygmomanometer inconvenient to handle and store during daily use. Moreover, all the existing electronic sphygmomanometers, whether they are MWD-based or MWI-based, are equipped with a controlling unit, a display, and several function buttons. For powering sophisticated measuring operations actuated by these function buttons and for powering the display to present related messages and data to the user, it is necessary for such an existing electronic sphygmomanometer to have a high-capacity battery, which further increases the overall volume of the sphygmomanometer and is adverse to microminiaturization.

As to its mechanical structure, such an electronic sphygmomanometer is usually designed with special considerations given to pressure and deflation. In order not to hinder deflation, the internal components of the sphygmomanometer are typically arranged around the axis of the air pump, causing them to be stacked at the radial plane of the air pump. This layout can disadvantageously increase the electronic sphygmomanometer in all dimensions. For example, the patent document numbered CN215457972U discloses a wrist type electronic sphygmomanometer, which comprises a face shell and a bottom shell. The face shell is equipped with an on-off key and an LCD display screen. The bottom shell at its bottom is formed with a battery room and a battery cover. A cavity is formed between the face shell and the bottom shell for receiving a circuit board and an inflation mechanism. The inflation mechanism comprises an air pump installed in the cavity, a 6-way silicone tube communicated with an air supply tube of the air pump, an electromagnetic valve communicated with the 6-way silicone tube, and an air supply tube joint of the wrist strap communicated with the 6-way silicone tube and extending to the bottom of the bottom shell. The electromagnetic valve has an its exhaust tube joint extending to the bottom of the bottom shell. Pressure sensors are arranged at the top of the 6-way silicone tube, close to the position of the connecting hole of the air pump, close to the position of the connecting hole of the electromagnetic valve and close to the position of the connecting tube joint of the wrist strap air supply tube, respectively.

There are also electronic sphygmomanometers in the prior art that are configured to work with smart terminals. For example, the patent document numbered CN203000916U provides a pedestal for an electronic sphygmomanometer, wherein the pedestal contains a Bluetooth communication module. However, the function of Bluetooth communication is not built in the electronic sphygmomanometer but comes from modular expansion. This fact makes the known sphygmomanometer bulky and less handy.

Another known electronic sphygmomanometer, as disclosed in CN112584763A, is equipped with the capability of wireless communication, and its capability in this term is almost equivalent to the device provided by CN203000916U because they both rely on modular expansion. Although this type of sphygmomanometer uses integrated modules, circuits contained therein are substantively separate each other, such as those chips and circuits for measuring and analyzing blood pressures and electrocardiographic patterns, an air pump unit for inflation (including a valve controlling unit), a communication module, and related controlling units. Such an electronic sphygmomanometer, while being rich in function, is far from being small and handy. In fact, it is more like a professional medical instrument design for trained medical staff than a home healthcare device intended for daily use.

There are also solutions in the art about associating an electronic sphygmomanometer with a smart terminal, but only to realize advance functions, such as user reminding, health data logging or transmission, at the smart terminal. Of course, there are some more sophisticated designs. For example, CN112334887A discloses a solution where image recognition is used to communicate manual contents and operational instructions.

There are also wearable sphygmomanometers like the device described in the patent document numbered CN107752997A. While the known electronic sphygmomanometer, for being wearable, is relatively small in size, it is questionable in terms of accuracy. This is because the prior-art device performs indirect pressurization by using a mechanical tightening means to compress a wrist collar that contains a preinflated airbag, instead of using an air pump for pressurization to achieve an oscillometric measurement. Specifically, the elastic material used to form the airbag and the air volume in the airbag are likely to have variations over time, and these variations are less predictable as these variables are highly subject to material elasticity but also environmental parameters, such as air temperature and pressure. As a result, not only is accurate measurement (not to mention medical-grade accuracy) a challenge, but there is risk of failure in measurement. All the electronic sphygmomanometer discussed previously are provided as a standalone device that operates with the display and operational buttons packed on its surface, and this highly-packed layout is usually a cause of poor user experience. Further sphygmomanometers are disclosed in US2007/167845A1, CN104188643Aand US2006/187481A1. Furthermore document CN103735258A discloses a system composed of a cuff, a sphygmomanometer, and a smart terminal, wherein the cuff and the sphygmomanometer are connected together, and the sphygmomanometer and the smart terminal are connected via Bluetooth.

### SUMMARY OF THE APPLICATION

To address the shortcomings of the prior art, the present application is made. It relates to medical instruments. More particularly, the present application relates to a sphygmomanometer, to a Bluetooth electronic sphygmomanometer, in particular to a sphygmomanometer based on a reversible air pump, and especially to an electronic sphygmomanometer using the MWI (measuring while inflating) method. The aim of the present application is to realize miniaturization and portability of sphygmomanometers while ensuring accurate measurement of blood pressure values.

The invention is defined by the appended claims.

The inventive sphygmomanometer comprises a main body and a cuff. The cuff contains therein an airbag that can be inflated and deflated. Preferably, the cuff has two forms of armband and wristband.

Further, the cuff defines a first connecting surface mechanically coupled to the main body and a second connecting surface configured to be wound around the wrist and arm of a to-be-measured object. The second connecting surface is integrally formed with the first connecting surface and is mechanically separated from the main body.

The main body primarily comprises a casing, a circuit board, a reversible air pump, a battery, and a switch installed on the surface of the casing. The circuit board integrates thereon a main controlling unit, a driving unit, a detecting unit, and a charging unit

The switch on the surface of the casing is only used to turn on/off the sphygmomanometer and switch Bluetooth working states. Except for the switch, all operating units of the sphygmomanometer related to measurement of blood pressure values are provided on a smart terminal that is bound with the sphygmomanometer by means of wireless communication. The smart terminal is configured to provide operational instructions to the sphygmomanometer, such as starting measurement, stopping measurement, and/or performing dynamic measurement.

The main controlling unit primarily comprises a controlling chip. The operating units as well as the displaying unit related to measurement are powered by the smart terminal bound with the sphygmomanometer by means of wireless communication. Instructions to the operating units and the displaying unit are transmitted and processed by the controlling chip. In summary, the chip has two main functions: enabling wireless communication with the smart terminal; and performing tasks related to measurement of blood pressure values. Hence, the controlling chip is a Bluetooth chip, particularly a Bluetooth chip of specifications of Bluetooth 4.0 or above that features low energy.

According to the invention the driving unit of the sphygmomanometer includes an inflation driver, a reversible air pump, and a driving circuit. The reversible air pump has in terms of its control signals exclusively electrically connected to the controlling chip. The reversible air pump has an airflow passage. The direction of an airflow passing through the airflow passage is determined by motor rotation directions. The airflow passage may be inflating passage and/or a deflating passage. The reversible air pump is configured to change its operating conditions according to variations of the polarity of the current flow in the driving circuit it is located in. The operating conditions include a first operating condition wherein the motor is driven to rotate in the forward direction so it inflates the cuff and a second operating condition wherein the motor is driven to rotate in the reverse direction so it deflates the cuff.

The inflation driver has in terms of its control signals exclusively electrically connected to the controlling chip, and is configured to activate and/or switch and/or end the working states of the reversible air pump according to the measurement instructions issued by the controlling chip.

According to the invention, the detecting unit of the sphygmomanometer comprises a pressure sensor installed on the circuit board and comprises an analog-to-digital converter as well as a detecting circuit. The pressure sensor has a detection air pipe that extends into the cuff through the detection air channel so as to acquire pressure signals. The pressure signals are analog voltage signals, which are converted into digital signals by the analog-to-digital converter before being sent to the controlling chip.

Preferably, the charging unit of the sphygmomanometer comprises a data interface, a battery together with a battery managing module, an interface circuit, and a charging circuit. The data interface is used to charge the battery of the sphygmomanometer via the interface circuit and the charging circuit. The battery is used to power the components of the sphygmomanometer. The battery managing module is used to provide the battery with reliable over-charge and/or over-discharge protection, over-current and/or over-heat protection and fault diagnosis. Particularly, the data interface may be further connected to other health-detecting devices in mechanical way and via signals, so as to obtain additional health-monitoring functions.

The battery is integrated in the main body, or is removably installed in the main body. In addition to power-consuming units of the battery itself and sensors related to the measurement, the sphygmomanometer only have the Bluetooth chip and the reversible air pump as power-consuming units thereof.

As to the mechanical structure, the casing of the inventive sphygmomanometer comprises a shell and an end cap, which jointly define some cavity therebetween. The cavity is shaped to the layout of internal components of the sphygmomanometer. The cavity in the present embodiment may be formed as a pump cavity for receiving the reversible air pump and a battery cavity for receiving the battery.

Preferably, the main body and the cuff are removably connected in a first arrangement where the axis of the reversible air pump is parallel to a third direction or in a second arrangement where the axis of the reversible air pump is parallel to a first direction.

In the first arrangement, the main body is perpendicularly mounted onto the first connecting surface of the cuff in the negative direction of then third direction. The components in the sphygmomanometer are tightly arranged along the third direction and around the reversible miniature air pump. In the second arrangement, the main body is mounted onto the first connecting surface of the cuff in the first direction connection. The components in the sphygmomanometer are tightly arranged along the first direction and around the reversible miniature air pump.

The beneficial effects of the present application are described in the following paragraph.

Most traditional electronic sphygmomanometers have a display integrated with the main body, and, for ensuring good legibility and integrity of the displayed information, the display must have a significant size, which means it takes a considerable area on the surface of the main body. In addition, these traditional electronic sphygmomanometers usually have multiple function buttons arranged on the surface of the main body, and a complete set of function buttons also occupies a large area in the surface of the main body. These jointly limit microminiaturization of the sphygmomanometers. As compared to the prior-art devices, the sphygmomanometer of the present application removes the display, and move the displaying unit and all the operating units related to measurement of blood pressure values except for the switch to an external smart terminal. In other words, control instructions to the sphygmomanometer given by a user from the smart terminal are wirelessly transmitted to the main body, and measurement results are then wirelessly transmitted to and displayed at the smart terminal. As such, design of the sphygmomanometer is released from restrictions coming with the necessity of arranging the display and the function buttons on the surface of the sphygmomanometer, thereby giving designer more opportunities to further simplify and optimize the internal structure of the sphygmomanometer.

Further, the inventive sphygmomanometer implements the MWI method, which is an oscillometric method. It is well known that oscillometric methods are the most reliable solution for non-invasive blood pressure measurement, and therefore measurement accuracy is secured with the inventive sphygmomanometer.

Moreover, the sphygmomanometer of the present application employs a reversible air pump that can be switched between inflation and deflation by simply changing the rotation direction of the motor. This allows the inventive sphygmomanometer to omit an electromagnetic valve used in traditional sphygmomanometers (i.e., sphygmomanometers using the MWD method) for deflation. Furthermore, since the sphygmomanometer of the present application is based on the MWI method and thus it detects the pressure in the period when the artery is pressed, the inventive device does not need any quick bleeding valve for quick deflation and pressure relief. As such, it is clear that the present application, by eliminating the need of an electromagnetic valve and a quick bleeding valve, reduces manufacturing costs, simplifies the internal structure of the electronic sphygmomanometer, and creates more opportunities for microminiaturization than traditional electronic sphygmomanometer.

On the basis of the configuration described previously, two exemplificative arrangements of the main body and the cuff are provided herein. The first one is that the main body is perpendicularly connected to the first connecting surface of the cuff in the third direction connection, and the second arrangement is that the main body is parallelly connected to the first connecting surface of the cuff in the first direction. Whether it is the first arrangement or the second arrangement, the internal components of the sphygmomanometer are closely arranged around the reversible air pump and in a certain direction. This means that the inventive sphygmomanometer is optimized in terms of internal structure design. Additionally, in the present application, the connection/disconnection between the main body and the cuff is direct and simple, so can be easily realized by directly plugging in/pulling out, making the inventive sphygmomanometer user-friendly. To sum up, the inventive sphygmomanometer implements a scientific method for measurement of blood pressure, eliminates abundant components, and optimize the internal structure. With the three-pronged strategy, the present application enables microminiaturization and portability while providing high measurement accuracy of a sphygmomanometer.

### BRIEF DESCRIPTION OF THE DRAWINGS

FIG. 1 is a block diagram illustrating hardware connection of a sphygmomanometer of the present application;
FIG. 2 is a perspective view of a sphygmomanometer in assembled state according to Embodiment 1 of the present application;
FIG. 3 is a view of the sphygmomanometer in preliminary disassembled state according to Embodiment 1 of the present application;
FIG. 4 is an exploded perspective view of the sphygmomanometer according to Embodiment 1 of the present application;
FIG. 5 is a perspective view of a sphygmomanometer in assembled state according to Embodiment 2 of the present application;
FIG. 6 is a view of the sphygmomanometer in preliminary disassembled state according to Embodiment 2 of the present application; and
FIG. 7 is an exploded perspective view of the sphygmomanometer according to Embodiment 2 of the present application.

### DETAILED DESCRIPTION OF THE APPLICATION

In this document, any directional expression related to any component is for the sake of reading convenience and by no means forms any limitation to the present application. It is also to be understood that, throughout the disclosure, spatial descriptions like "central", "longitudinal", "transverse", "up", "down", "left", "right", "vertical", "horizontal", "inner" and "outer" are indicated with respect to the orientation or locational relationship shown in the figures, and are only for laconic descriptions of relevant components, without indicating or implying that the described devices or components must have any certain orientation, or be constructed or operated in any certain orientation, so they shall never be construed as any limitation to the present application. In addition, any ordinal number such as "first" or "second" used herein is merely for differentiating clearly a claimed element having a certain name from another claimed element having the same name, and has no ordinal or technical significance. Unless otherwise specified, as used herein, "connection" and "coupling" both include direct and indirect connection (coupling).

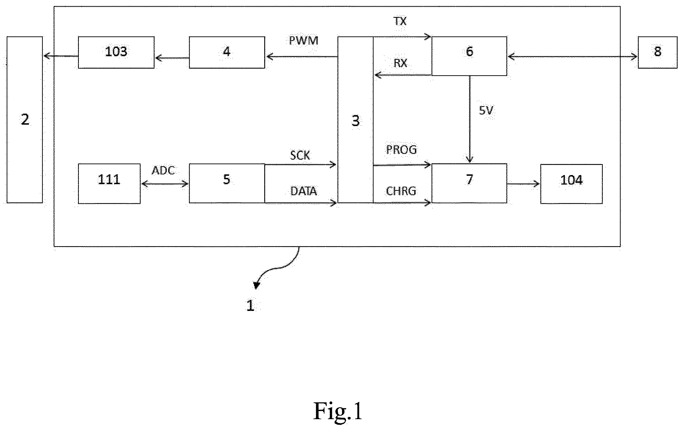

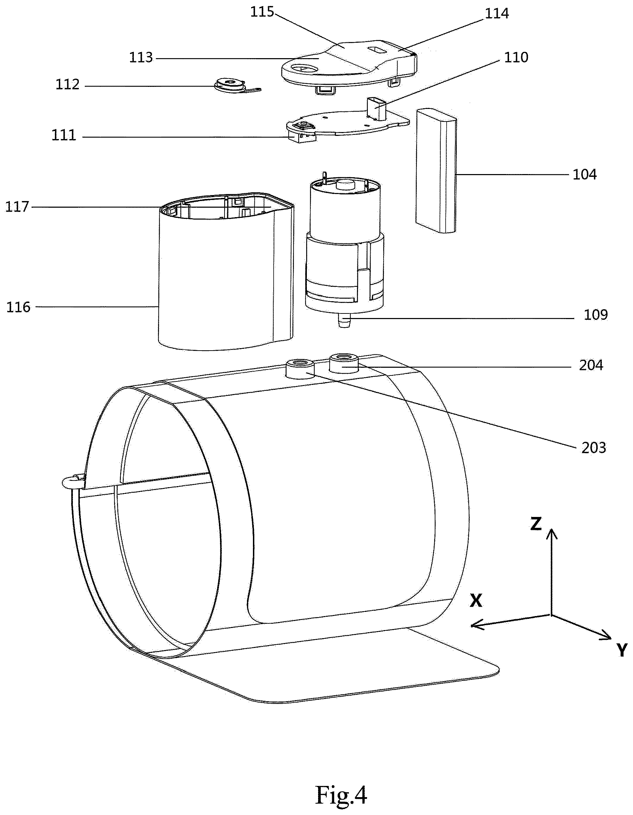

The present application relates to a sphygmomanometer, and particularly to a Bluetooth electronic sphygmomanometer featuring a reversible air pump. The sphygmomanometer involves technical contents in terms of mechanical structure and hardware structure. Referring to FIG. 1 to FIG. 7, as to its mechanical structure, the inventive sphygmomanometer has a main body 1 that primarily comprises a casing, a circuit board 102, a reversible air pump 103, a battery 104, and a switch 112 installed on the surface of the main body 1. As to its hardware structure, the inventive sphygmomanometer integrates therein a main controlling unit, a driving unit, a detecting unit, and a charging unit.

As to hardware, according to the invention, as shown in FIG. 1 to FIG. 7, the main controlling unit in the present application comprises a Bluetooth chip 3 as its controlling chip. Preferably, the Bluetooth chip 3 is a Bluetooth chip of specifications of Bluetooth 4.0 or above that features low energy. The Bluetooth chip 3 is used to establish wireless communication with the smart terminal and to perform controlling, computing, and analyzing tasks related to measurement of blood pressure values. The operating units and the displaying unit related to measurement of blood pressure values are installed on the smart terminal. The switch installed on the surface of the main body 1 is exclusively used to turn on/off the sphygmomanometer and switch working states of the Bluetooth chip 3. The Bluetooth chip 3 receives and acts on operational instructions related to blood pressure measurement from the smart terminal. Therein, the operational instructions may include activating measurement, stopping measurement, and/or performing dynamic measurement.

The driving unit of the inventive sphygmomanometer comprises an inflation driver, a reversible air pump 103, and a driving circuit 4. The reversible air pump 103 and the inflation driver are connected to each other and both electrically connected to the Bluetooth chip 3. The inflation driver is preferably a motor driver. The inflation driver is configured to control the working states of the reversible air pump according to measurement instructions issued by the Bluetooth chip 3, so as to, for example, turn on/off the reversible air pump, or switch the reversible air pump between working modes. The airflow passage of the reversible air pump 103 is switchable between two modes, namely the inflating passage mode and the deflating passage mode, according to the forward or reverse rotation of the motor. In other words, the reversible air pump 103 can change between operating conditions with variations of the polarity of the current flow in its driving circuit 4. Specifically, when the motor is driven to rotate in the forward direction, the reversible air pump 103 is in the first operating condition wherein it inflates the cuff, and when the motor is driven to rotate in the reverse direction, the second operating condition is in the second operating condition wherein it deflates the cuff. For example, an air pump disclosed in the patent document numbered 217682211U comprises an inflating passage formed by an airbag, a buffering air chamber and an air inlet, and a deflating passage formed by an air outlet and an air inlet connected together. When the motor rotates forward, the airflow enters the cuff through the air inlet. At this time the air pump is in the inflation operating condition. When the motor rotates in the reverse direction, the airflow goes out of the cuff through the air outlet. At this time the air pump is in the deflation operating condition. Control the motor of the reversible air pump 103 for forward or reverse rotation can be accomplished by adjusting the polarity of the current flow in the driving circuit 4. Since circuit structures having this feature are well known in the art, details related thereto are omitted herein for conciseness.

In the present application, the detecting unit comprises a pressure sensor 111 mounted on the circuit board 102, an analog-to-digital converter, and a detecting circuit 5. The pressure sensor 111 has a detection air pipe passing through the detection air channel and extending into the cuff to acquire pressure signals. The pressure signals are analog voltage signals that are to be converted into digital signals by an analog-to-digital converter before sent to the Bluetooth chip 3.

In the present application, the charging unit comprises a data interface 110, a battery 104, a battery managing module, an interface circuit 6, and a charging circuit 7. The data interface 110 is used to charge the battery 104 of the sphygmomanometer through the interface circuit 6 and the charging circuit 7. The battery 104 is used to power the various components of the sphygmomanometer. The battery managing module is used to provide the battery 104 with reliable over-charging and/or over-discharge protection, over-current and/or over-heat protection, and fault diagnosis. Particularly, data interface 110 may further be connected to another health monitoring device (such as a plug-in 8) in terms of mechanical connection and/or signal connection so as to obtain more health-monitoring functions.

Preferably, the battery 104 of the sphygmomanometer may be integrated in the main body 1. Alternatively, the battery 104 may be removably installed in the main body 1. In an alternative mode, in addition to power-consuming units of the battery 104 itself and sensors related to the measurement, the sphygmomanometer only have the Bluetooth chip 3 and the reversible air pump 103 as power-consuming units thereof.

It is to be noted that since the Bluetooth chip 3 serves for not only communication but also operations related to blood pressure measurement, and the motor has to be driven to rotate forward or reversely for the reversible air pump 103 to accomplish inflation or deflation, they are jointly responsible for most power consumption of the sphygmomanometer, so it is arguable that the Bluetooth chip 3 and the reversible air pump 103 jointly define how power-consuming the sphygmomanometer is. Of course, the managing module of battery 104 itself and the pressure sensor 111 for blood pressure measurement, and the analog-to-digital converter, and even the Bluetooth antenna all consume energy, but their aggregative energy consumption is at least one order, or even several orders, of magnitude smaller than that of the combination of the Bluetooth chip 3 and the reversible air pump 103. In this case, it is arguable that the only two meaningful power-consuming units in the inventive sphygmomanometer are the Bluetooth chip 3 and the reversible air pump 103.

According to one preferred mode, as shown in FIG. 1, the Bluetooth chip for control, computation, and communication has the following connection arrangements.

The RX pin and the TX pin of the Bluetooth chip 3 are connected to the interface circuit 6. When the data interface 110 is connected to an external power source, the RX pin and the TX pin realize level shifting for charging. When the data interface 110 is connected to other health monitoring devices (such as a plug-in 8), the RX pin and the TX pin are used to establish communication with these devices.

Further, the interface circuit 6 leads power into the charging circuit 7 so the charging circuit 7 can charge the battery 104. The charging circuit 7 is connected to the Bluetooth chip 3 through the PROG pin and the CHG pin of the Bluetooth chip 3, so that the charging circuit 7 can have a constant charging current under the control of the Bluetooth chip 3 and that the Bluetooth chip 3 can monitor the charging circuit 7. The Bluetooth chip 3 is connected to the detecting circuit through the SCK pin and the DATA pin, and then connected to the pressure sensor 111 so as to acquire air pressure variations during the MWI method. The Bluetooth chip 3 is connected to the driving circuit 4 through the PWM pin so as to drive the reversible air pump 103 to inflate and/or deflate the cuff 2 and control and hold the inflation speed stable.

More preferably, the remaining pins of the Bluetooth chip 3 left idle may be connected to a plug-in managing circuit for managing external health monitoring devices (such as a plug-in 8), and further connected to a mobile APP through BLE so as to form a plug-in measurement and management system.

The technical contents of the hardware structure of the inventive sphygmomanometer have been described and the mechanical structure of the sphygmomanometer will be further detailed below with reference to the accompanying drawings.

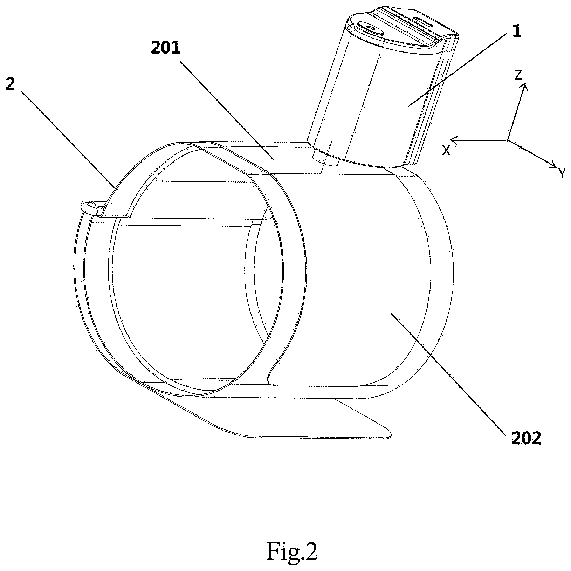

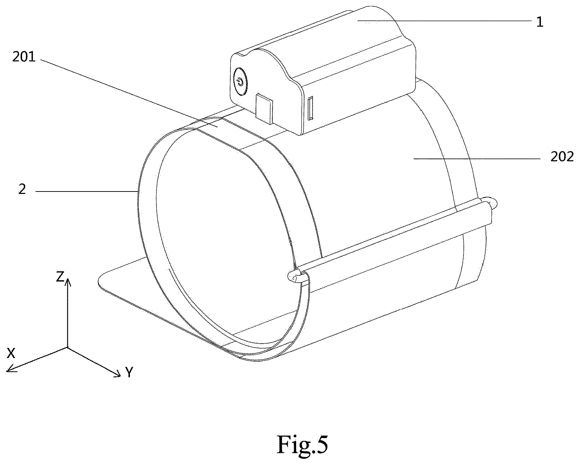

As shown in FIG. 2 and FIG. 5, the inventive sphygmomanometer primarily comprises a main body 1 and a cuff 2. The cuff 2 is provided therein with an airbag (not shown) that can expand and contract as it is inflated and deflated by an air pump. The cuff 2 may be formed as an armband or a wristband to as to allow blood pressure measurement at the arm or the wrist of a user. Further, the cuff 2 defines a first connecting surface 201 mechanically coupled to the main body 1 and a second connecting surface 202 configured to be wound around the wrist and arm of a to-be-measured object. Particularly, the second connecting surface 202 is integrally formed with the first connecting surface 201 and is mechanically separated from the main body 1.

For convenient description, the direction in which the cuff 2 is worn onto the arm of the user is defined as the first direction; the direction that is parallel to the first connecting surface of the cuff 2 and perpendicular to the first direction is defined as the second direction; and the normal direction that is perpendicular to the first connecting surface of the cuff 2 is defined as the third direction. When placed into the X-Y-Z coordinate system as shown in FIG. 2 to FIG. 7, the first direction, the second direction, and the third direction are positive directions of the X axis, the Y axis, and the Z axis, respectively, and the reverse directions of the first direction, the second direction, and the third direction are negative directions of the X axis, the Y axis, and the Z axis, respectively.

As shown in FIG. 2 and FIG. 5, the main body 1 comprises a casing, a circuit board 102, a reversible air pump 103, a battery 104, and a substrate 105. Depending on the assembling alternatives and connection directions between the main body 1 and the cuff 2, the main body and the cuff may be alternatively assembled into the first arrangement or the second arrangement. Specifically, in the first arrangement, the reversible air pump has its axial direction parallel to the third direction, and in the second arrangement, the reversible air pump has its axial direction parallel to the first direction. Correspondingly, the internal components in the main body 1 are arranged closely along the first direction or the third direction, depending on the arrangement.

The casing of the main body 1 may be composed of a shell 101 and an end cap 106. Specifically, the shell 101 and the end cap 106 are fixedly combined. In an alternative mode, the shell 101 and the end cap 106 may be detachably assembled. Depending on the arrangement of the main body 1 and the cuff 2, the shell 101 may have different contours so as to correspondingly accommodate the other components arranged in the main body 1, as detailed below with reference to two embodiments.

### Embodiment 1

According to one preferred mode, as shown in FIG. 1, the main body 1 extends along the third direction and is basically perpendicularly inserted onto the first connecting surface 201 of the cuff 2, which means that the installed reversible air pump 103 of the sphygmomanometer has its axial direction in accordance with the third direction. Correspondingly, the components inside the main body 1 are all arranged closely in the third direction.

As shown in FIG. 2, the shell 101, the end cap 106, and the substrate 105 of the sphygmomanometer jointly define an accommodating cavity for receiving the reversible air pump 103, the battery 104, and the circuit board 102. The reversible air pump 103 is perpendicularly installed on the substrate 105 in the positive direction of the third direction, and has a circuit board 102 located at its end surface far from the substrate 105. The battery 104 is installed between the substrate 105 and the circuit board 102. The pressure sensor 111 may be installed on the surface of the circuit board 102 close to the reversible air pump 103. A detection air pipe (not shown) extends from the pressure sensor 111 into the cuff through the detection air channel 107 so as to acquire pressure signals. The detection air channel 107 is located between the substrate 105 and the circuit board 102, and juts out form the substrate 105 to form a detection air channel connector 108.

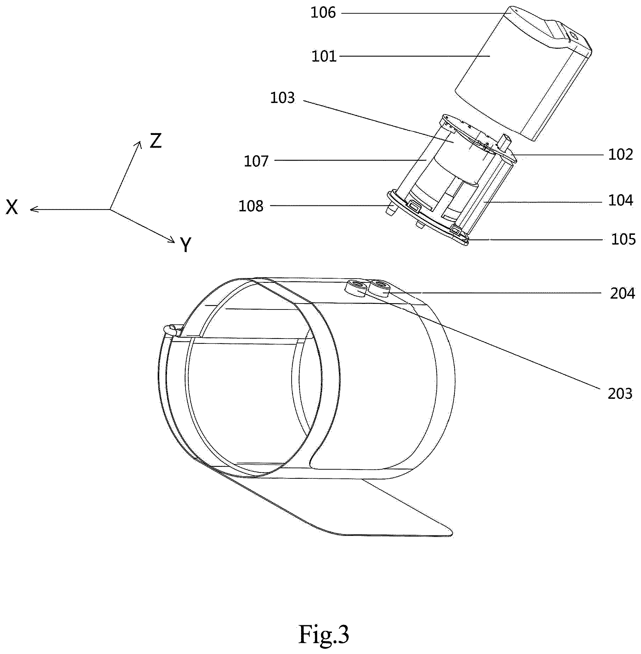

As shown in FIG. 2 and FIG. 3, the valve 109 of the reversible air pump 103 extends in the negative direction of the third direction and juts out of the substrate 105, thereby getting connected with the valve socket 204 on the first connecting surface 201 of the cuff 2 so that the reversible air pump 103 can perform inflation and deflation on the cuff 2. Further, the first connecting surface 201 of the cuff 2 is further provided with a detection air channel joint 203 that matches the detection air channel connector 108. The location of the detection air channel joint 203 is aligned with the site where the detection air channel connector 108 juts out of the substrate 105.

According to one preferred mode, the circuit board 102 is provided with a data interface 110 at its side close to the battery 104 and the data interface 110 is arranged in the positive direction of the third direction. The end cap 106 is further provided with a switch 112. The data interface 110 is used to charge the sphygmomanometer or is connected by wires to a terminal for wired data transmission. Further, the data interface 110 is a port made according to the USB specifications, such as a mini-USB port, a micro-USB port, or a Type-C port, wherein a Type-C port is most preferred.

The circuit board is essentially a circuit board integrating numerous electronic units thereon. In the present embodiment, the circuit board 102 incorporates a main controlling unit, a computing and analyzing unit, a communicating unit, and an analog-to-digital converter. The button 111 and the reversible air pump 103 are both in communicative connection with the main controlling unit. The main controlling unit receives the instruction made through the button 111 and then sends intermediate instruction to the reversible air pump 103. The reversible air pump 103, according to the instruction issued by the main controlling unit, performs inflation or deflation. The analog-to-digital converter receives the pressure data measured by the pressure sensor and converts the data into electrical signals to be sent to the computing and analyzing unit. The computing and analyzing unit process the signals obtained through analog-to-digital conversion so as to get the final detection results. The results are then wirelessly sent to the mobile terminal through the communicating unit. Alternatively, the final detection results generated by the computing and analyzing unit through data processing may be transmitted by wires to the mobile terminal through the data interface 110. Preferably, the computing and analyzing unit may be in communicative connection with the data interface 110 by means of asynchronous communication (URAT).

As shown in FIG. 1, FIG. 2, and FIG. 3, the shell 101 of the sphygmomanometer is configured as a hollow tube that has a cross-section shaped like a closed U. The shell 101 is shaped and sized to the contours of the reversible air pump 103 and the battery 104. The curved lateral 116 of the shell 101 forms a pump cavity matching the reversible air pump 103 in shape, and the rectangular lateral 117 of the shell 101 forms a battery cavity matching the battery 104 in shape. The curved lateral 116 of the shell 101 well fits the palm of the user, so that when the user holds the sphygmomanometer, the bending fingers of the user can naturally rest on the rectangular lateral 117. This provides the fingers with proper support and facilitates operations of the user for assembling or dissembling the main body 1 and the cuff 2.

As shown in FIG. 2 and FIG. 3, the end cap 106 is located at the side far from the substrate 105 and is detachably attached to the shell 101. The surface of the end cap 106 is formed by a first plane 113, a second plane 114, and a connecting incline 115 connecting the former two. A height difference exists between the first plane 113 and the second plane 114 in the third direction. For flushing the edge of the data interface 110 with the edge of the rectangular opening on the second plane 114, the height difference shall be equal to the height of the data interface 110 installed on the circuit board 102. The height difference so designed also reduces the altitude of the first plane 113 in the third direction, thereby further reducing the axial size of the casing of the sphygmomanometer. Moreover, for preventing formation of a right-angled connection between the first plane 113 and the second plane 114 due to steep transition therebetween, the connecting incline 115 having a predetermined inclination is provided between the first plane 113 and the second plane 114 so that the second plane 114 can smooth transit to the first plane 113. Preferably, the predetermined inclination is between 15° and 30°.

### Embodiment 2

As a further improvement on Embodiment 1, in the present embodiment, the main body 1 extends along the first direction and is roughly parallelly inserted onto the first connecting surface 201 of the cuff 2, as shown in FIG. 5. In other words, the installed reversible air pump 103 of the sphygmomanometer has an axial direction in accordance with the first direction. As compared to Embodiment 1, the present embodiment lowers the center of gravity of the installed main body 1 and increases the contact area between the main body 1 and the cuff 2, thereby further stabilizing the connection between the main body 1 and the cuff 2.

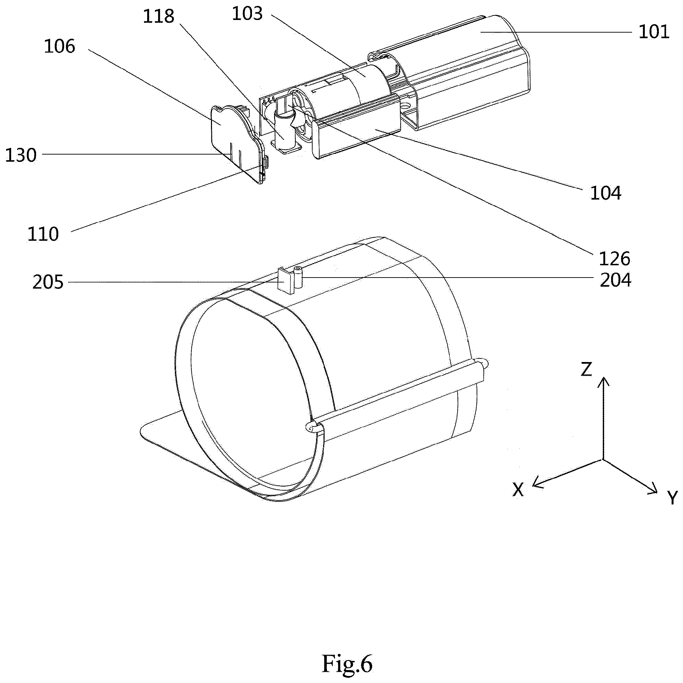

According to one preferred mode, as shown in FIG. 5 and FIG. 6, the main body 1 is primarily composed of a shell 101, an end cap 106, a three-way pipe 118, a reversible air pump 103, and a battery 104. Since the main body 1 is arranged in the first direction, the reversible air pump 103 can be directly fixed to the inner wall of the casing and the inner wall of the end cap 106, instead of having to be supported by the substrate 105. Correspondingly, the remaining components of the main body 1 are arranged closely around the reversible air pump 103 and in the first direction, as detailed below.

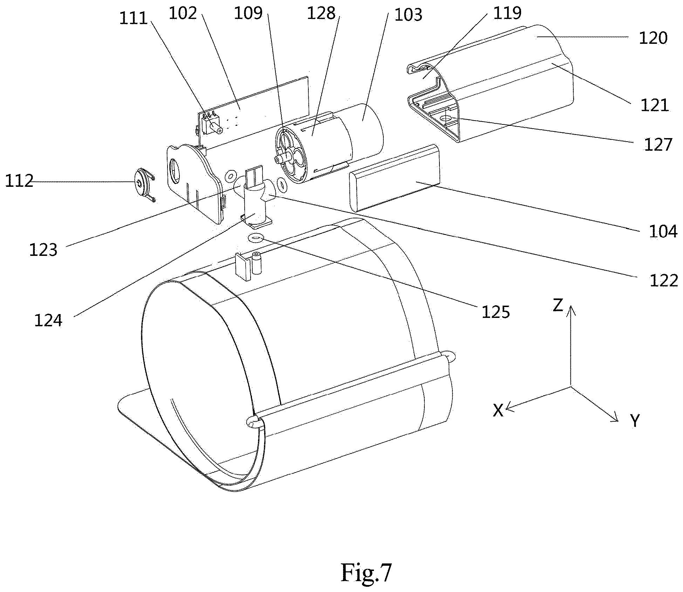

As shown in FIG. 6 and FIG. 7, in the first direction, the end cap 106, the three-way pipe 118, the reversible air pump 103, and the shell 101 are arranged coaxially. The circuit board 102 and the battery 104 are arranged opposite to each other at the periphery of the reversible air pump 103. The shell 101 is open at one end and closed at the opposite end. The open end is configured to be combined with the end cap 106 so as to define therebetween a closed cavity. Further, the surface of the shell 101 is formed by a curved part and a rectangular part connected together, so that the cavity is divided into three parts in the second direction, which are a board cavity 119 for accommodating the circuit board 102, a pump cavity 120 for accommodating the reversible air pump 103, and a battery cavity 121 for accommodating the battery 104.

According to one preferred mode, the reversible air pump 103 has its axial direction parallel to the first direction and has its outer peripheral surface provided with a retaining sleeve 128 that is adapted to a retaining groove 127 formed on the inner wall of the shell 101 so that the reversible air pump 103 can be fixed in the cavity. Further, at the outer peripheral side of the reversible air pump 103, the battery 104 and the circuit board 102 are arranged opposite to each other and in the first direction. They are fixed in the battery cavity 121 and in the board cavity 119 of the casing, respectively.

As shown in FIG. 7, the valve 109 of the reversible air pump 103 is connected to the first port 122 of the three-way pipe 118 in the first direction and secured from leakage by a seal ring 125. The pressure sensor 111 is installed on the circuit board 102 at the side close to the end cap 106 in the first direction. The three-way pipe 118 has its second port 123 extends in the negative direction of the second direction to the pressure sensor 111 of the circuit board 102. The third port 124 extends in the negative direction of the third direction and is connected to the valve socket 109 on the first connecting surface 201 of the cuff 2. The seal ring 125 then seals the second port 123 and the third port 124 of the three-way pipe 118.

As such, the three-way pipe 118 and the valve 109 of the reversible air pump 103 form an airflow passage and a detection passage. The airflow passage is formed by the valve 109, the first port 122, and the third port 124, so that the reversible air pump 103 can smoothly inflate and deflate the cuff 2. The detection passage may be formed by the pressure sensor 111, the second port 123, and the third port 124. Therein, the detection air pipe (not shown) of the pressure sensor 111 extends from the detection passage into the cuff so as to acquire pressure signals. Further, the three-way pipe 118 has retaining plates 126 on both the end in the third direction and the end of the third port 124. Correspondingly, matching retaining grooves 127 are formed on the inner wall of the casing, so that the three-way pipe 118 can be secured on the inner wall of the casing.

Thus, the three-way pipe 118 connects the valve 109 of the reversible air pump 103 to the cuff 2, so that the airflow passage can turn from the first direction, in which the valve 109 extends, to the third direction. This well addresses the problem how the cuff 2 can be inflated when the main body 1 is arranged in the first direction arrangement.

As shown in FIG. 6 and FIG. 7, the data interface 110 is provided on the end cap 106 at the side far from the circuit board 102 in the second direction. The switch 112 is located in a round through hole preformed in the end cap 106 and is in mechanical contact with the button 111. For further ensuring firm connection between the third port 124 of the three-way pipe 118 and the valve socket 109 of the cuff 2, the end cap 106 is formed at its surface with a retaining socket 129, which is shaped and located to be adapted to the retaining insert 205 on the first connecting surface 201 of the cuff 2. In the present embodiment, the circuit board 102 and the data interface 110 have similar structures and functions to their counterparts as described in Embodiment 1, and repeated description is herein omitted.

The traditional electronic sphygmomanometers using the MWD method collect data in the process of deflation, and therefore need an electromagnetic valve for enabling deflation as well as a mechanical or electromagnetic quick bleeding valve for controlling the speed of deflation. Differently, the inventive sphygmomanometer uses the MWI method and thus can simply collect pressure data while inflating. This eliminates the need of a quick bleeding valve for quick deflation and pressure relief. Additionally, since the reversible air pump 103 is capable of inflation and deflation itself, there is no need for an additional electromagnetic valve for the purpose of deflation. This significantly reduces manufacturing costs while preventing complicity of internal structure due to redundant components of the sphygmomanometer, thereby allowing further microminiaturization of the sphygmomanometer. Moreover, in the present application, the main body 1 and the cuff 2 are connection in a simple way, so the user can assemble or dissemble the components through easy insertion and pull. This significantly facilitates handling and storage of the sphygmomanometer, thereby improving the sphygmomanometer in portability.

## Claims

1. A sphygmomanometer configured to be paired with a smart terminal, comprising a main body (1) equipped with a circuit board (102), a reversible air pump (103), a single switch (112) and further comprising a cuff (2),
wherein the switch (112) is only used to turn on/off the sphygmomanometer and switch Bluetooth working states,
wherein the smart terminal is configured to provide operational instructions to the sphygmomanometer, such as starting measurement, stopping measurement, and/or performing dynamic measurement, given by a user from the smart terminal and wirelessly transmitted to the main body (1), and measurement results are then wirelessly transmitted to and displayed at the smart terminal,
wherein the main body (1) is integrated therein with the reversible air pump (103), wherein the reversible air pump (103) is configured to be removably pneumatically connected to the cuff (2) so as to establish air communication therewith,
wherein the reversible air pump (103) is electrically connected to a controlling chip via a driving circuit (4) for receiving control signals in the sphygmomanometer,
wherein the driving circuit (4) for driving the reversible air pump (103) is electrically connected to or integrated in the circuit board (102), and the driving circuit (4) is, in terms of control signals thereof for switching working modes, exclusively electrically connected to the controlling chip,
wherein the main body (1) primarily comprises a casing, the circuit board (102), the reversible air pump (103), a battery (104), and the switch (112) installed on the surface of the casing, the circuit board (102) integrates thereon a main controlling unit, a driving unit comprising the reversible air pump (103) and the driving circuit (4), a detecting unit, and a charging unit,
wherein the main controlling unit comprises the controlling chip in form of a Bluetooth chip (3),
wherein the Bluetooth chip (3) is configured to establish wireless communication with the smart terminal and to perform controlling, computing, and analyzing tasks related to measurement of blood pressure values, and to receive and act on operational instructions related to blood pressure measurement from the smart terminal, and wherein the detecting unit comprises a pressure sensor (111) mounted on the circuit board (102), an analog-to-digital converter, and a detecting circuit (5),
wherein the pressure sensor (111) has a detection air pipe passing through a detection air channel and configured to extend into the cuff (2) to acquire pressure signals in the form of analog voltage signals,
wherein the analog-to-digital converter is configured to convert the analog voltage signals into digital signals before sending the digital signals to the Bluetooth chip (3) via the detecting circuit (5).

2. The sphygmomanometer of claim 1, wherein the operating units and the displaying unit are all powered by the smart terminal.

3. The sphygmomanometer of claim 1 or 2, wherein the Bluetooth chip (3) is a Bluetooth chip (3) of specifications of Bluetooth 4.0 or above that features low energy.

4. The sphygmomanometer of any one of claims 1 to 3, wherein the reversible air pump (103) has an airflow passage that changes direction of airflow passing therethrough according to rotation directions of a motor of the reversible air pump (103).

5. The sphygmomanometer of any one of claims 1 to 4, wherein the cuff (2) contains therein an airbag that can be inflated and deflated, the cuff (2) defines a first connecting surface mechanically coupled to the main body (1) and a second connecting surface configured to be wound around the wrist and arm of a to-be-measured object.

## Patentansprüche

1. Ein Blutdruckmessgerät, das zur Kopplung mit einem Smart-Terminal ausgelegt ist, mit einem Hauptkörper (1), der mit einer Leiterplatte (102), einer umkehrbaren Luftpumpe (103) und einem einzigen Schalter (112) ausgestattet ist, sowie mit einer Manschette (2),
wobei der Schalter (112) ausschließlich zum Ein- und Ausschalten des Blutdruckmessgeräts sowie zum Umschalten von Bluetooth-Betriebszuständen dient,
wobei das Smart-Terminal so ausgelegt ist, dass es dem Blutdruckmessgerät Betriebsanweisungen erteilt, wie beispielsweise das Starten einer Messung, das Beenden einer Messung und/oder das Durchführen einer dynamischen Messung, wie sie von einem Benutzer über das Smart-Terminal eingegeben und drahtlos an den Hauptkörper (1) übertragen werden, und Messergebnisse anschließend drahtlos an das Smart-Terminal übertragen und dort angezeigt werden,
wobei der Hauptkörper (1) mit der umkehrbaren Luftpumpe (103) integriert ist,
wobei die umkehrbare Luftpumpe (103) so ausgelegt ist, dass sie abnehmbar pneumatisch mit der Manschette (2) verbunden werden kann, um eine Luftverbindung mit dieser herzustellen,
wobei die umkehrbare Luftpumpe (103) über eine Ansteuerschaltung (4) elektrisch mit einem Steuerchip verbunden ist, um Steuersignale im Blutdruckmessgerät zu empfangen,
wobei die Ansteuerschaltung (4) zum Ansteuern der reversiblen Luftpumpe (103) elektrisch mit der Leiterplatte (102) verbunden oder in diese integriert ist und die Ansteuerschaltung (4) hinsichtlich ihrer Steuersignale zum Umschalten der Betriebsmodi ausschließlich elektrisch mit dem Steuerchip verbunden ist,
wobei der Hauptkörper (1) im Wesentlichen ein Gehäuse, die Leiterplatte (102), die umkehrbare Luftpumpe (103), eine Batterie (104), sowie den auf der Oberfläche des Gehäuses angebrachten Schalter (112) umfasst, wobei auf der Leiterplatte (102) eine Hauptsteuereinheit, eine die umkehrbare Luftpumpe (103) und die Ansteuerschaltung (4) umfassende Ansteuereinheit, eine Erfassungseinheit und eine Ladeeinheit integriert sind,
wobei die Hauptsteuereinheit den Steuerchip in Form eines Bluetooth-Chips (3) umfasst,
wobei der Bluetooth-Chip (3) so konfiguriert ist, dass er eine drahtlose Kommunikation mit dem Smart-Terminal herstellt und Steuerungs-, Rechen- und Analyseaufgaben im Zusammenhang mit der Messung von Blutdruckwerten ausführt sowie Betriebsanweisungen im Zusammenhang mit der Blutdruckmessung vom Smart-Terminal empfängt und entsprechend reagiert, und
wobei die Erfassungseinheit einen auf der Leiterplatte (102) montierten Drucksensor (111), einen Analog-Digital-Wandler und eine Erfassungsschaltung (5) umfasst,
wobei der Drucksensor (111) eine Messluftleitung aufweist, die durch einen Messluftkanal verläuft und so ausgebildet ist, dass sie sich in die Manschette (2) erstreckt, um Drucksignale in Form von analogen Spannungssignalen zu erfassen,
wobei der Analog-Digital-Wandler so ausgelegt ist, dass er die analogen Spannungssignale in digitale Signale umwandelt, bevor die digitalen Signale über die Erfassungsschaltung (5) an den Bluetooth-Chip (3) gesendet werden.

2. Blutdruckmessgerät nach Anspruch 1, wobei die Bedieneinheiten und die Anzeigeeinheit alle über das Smart-Terminal mit Strom versorgt werden.

3. Blutdruckmessgerät nach Anspruch 1 oder 2, wobei der Bluetooth-Chip (3) ein Bluetooth-Chip (3) gemäß den Spezifikationen von Bluetooth 4.0 oder höher ist, der sich durch geringen Energieverbrauch auszeichnet.

4. Blutdruckmessgerät nach einem der Ansprüche 1 bis 3, wobei die umkehrbare Luftpumpe (103) einen Luftströmungskanal aufweist, der die Richtung des durch ihn hindurchströmenden Luftstroms entsprechend den Drehrichtungen eines Motors der umkehrbaren Luftpumpe (103) ändert.

5. Blutdruckmessgerät nach einem der Ansprüche 1 bis 4, wobei die Manschette (2) einen Luftbeutel enthält, der aufgeblasen und entleert werden kann, wobei die Manschette (2) eine erste Verbindungsfläche, die mechanisch mit dem Hauptkörper (1) verbunden ist, und eine zweite Verbindungsfläche aufweist, die so ausgebildet ist, dass sie um das Handgelenk und den Arm eines zu messenden Objekts gewickelt werden kann.

## Revendications

1. Sphygmomanomètre configuré pour être apparié avec un terminal intelligent, comprenant un corps principal (1) équipé d'une carte de circuit (102), d'une pompe à air réversible (103), d'un interrupteur unique (112) et comprenant en outre un brassard (2),
dans lequel l'interrupteur (112) est utilisé uniquement pour allumer/éteindre le sphygmomanomètre et commuter les états de fonctionnement Bluetooth,
dans lequel le terminal intelligent est configuré pour fournir des instructions de fonctionnement au sphygmomanomètre, telles que le démarrage de la mesure, l'arrêt de la mesure, et/ou la réalisation d'une mesure dynamique, données par un utilisateur depuis le terminal intelligent et transmises sans fil au corps principal (1), et les résultats de mesure sont ensuite transmis sans fil au terminal intelligent et affichés sur celui-ci,
dans lequel la pompe à air réversible (103) est intégré au corps principal (1),
dans lequel la pompe à air réversible (103) est conçue pour être connectée pneumatiquement de manière amovible au brassard (2) de manière à établir une communication d'air avec celui-ci,
dans lequel la pompe à air réversible (103) est connectée électriquement à une puce de commande par l'intermédiaire d'un circuit d'attaque (4) pour recevoir des signaux de commande dans le sphygmomanomètre,
dans lequel le circuit d'attaque (4) pour entraîner la pompe à air réversible (103) est électriquement connecté à ou intégré dans la carte de circuit (102), et le circuit d'attaque (4) est, en termes de signaux de commande de celui-ci pour commuter les modes de fonctionnement, exclusivement connecté électriquement à la puce de commande,
dans lequel le corps principal (1) comprend principalement un boîtier, la carte de circuit (102), la pompe à air réversible (103), une batterie (104) et l'interrupteur (112) installé sur la surface du boîtier, la carte de circuit (102) intègre sur celle-ci une unité de commande principale, une unité d'entraînement comprenant la pompe à air réversible (103) et le circuit d'attaque (4), une unité de détection et une unité de charge,
dans lequel l'unité de commande principale comprend la puce de commande sous la forme d'une puce Bluetooth (3),
dans lequel la puce Bluetooth (3) est configurée pour établir une communication sans fil avec le terminal intelligent et pour effectuer des tâches de commande, de calcul et d'analyse liées à la mesure de valeurs de pression artérielle, et pour recevoir et exécuter des instructions opérationnelles liées à la mesure de pression artérielle provenant du terminal intelligent, et
dans lequel l'unité de détection comprend un capteur de pression (111) monté sur la carte de circuit (102), un convertisseur d'analogique à numérique, et un circuit de détection (5),
dans lequel le capteur de pression (111) comporte un tuyau d'air de détection traversant un canal d'air de détection et conçu pour s'étendre dans le brassard (2) afin d'acquérir des signaux de pression sous la forme de signaux de tension analogiques,
dans lequel le convertisseur d'analogique à numérique est conçu pour convertir les signaux de tension analogiques en signaux numériques avant d'envoyer les signaux numériques à la puce Bluetooth (3) par l'intermédiaire du circuit de détection (5).

2. Sphygmomanomètre selon la revendication 1, dans lequel les unités de commande et l'unité d'affichage sont toutes alimentées par le terminal intelligent.

3. Sphygmomanomètre selon la revendication 1 ou 2, dans lequel la puce Bluetooth (3) est une puce Bluetooth (3) de spécifications Bluetooth 4.0 ou supérieures qui présente une faible consommation d'énergie.

4. Sphygmomanomètre selon l'une quelconque des revendications 1 à 3, dans lequel la pompe à air réversible (103) comporte un passage d'écoulement d'air qui change la direction de l'écoulement d'air le traversant en fonction des directions de rotation d'un moteur de la pompe à air réversible (103).

5. Sphygmomanomètre selon l'une quelconque des revendications 1 à 4, dans lequel le brassard (2) contient à l'intérieur une poche à air qui peut être gonflée et dégonflée, le brassard (2) définit une première surface de connexion couplée mécaniquement au corps principal (1) et une seconde surface de connexion conçue pour être enroulée autour du poignet et du bras d'un objet à mesurer.
